# EUROPÄISCHE PATENTSCHRIFT

(11) **EP 1 860 111 B1**
(45) Veröffentlichungstag und Bekanntmachung des Hinweises auf die Patenterteilung: **15.12.2010**
(21) Anmeldenummer: 07106994.2
(22) Anmeldetag: 26.04.2007
(51) Int. Cl.: C07D 495/04

(54) **Verfahren zur Stabilisierung von Thiophenderivaten**
Process for stabilising thiophene derivatives
procédé pour la stabilisation des derivés de thiophène

(30) Priorität: 04.05.2006 DE 102006020744
(43) Veröffentlichungstag der Anmeldung: 28.11.2007
(73) Patentinhaber: H.C. Starck Clevios GmbH, 38642 Goslar (DE)
(72) Erfinder: Jonas, Friedrich, 52066, Aachen (DE); Wussow, Klaus, 57250, Netphen (DE); Reuter, Knud, 47800, Krefeld (DE)
(74) Vertreter: Herzog, Martin

(56) Entgegenhaltungen:
- EP-A1- 1 142 888

## Beschreibung

Die Erfindung betrifft ein Verfahren zur Stabilisierung von Thiophenderivaten sowie nach diesem Verfahren erhältliche stabilisierte Thiophenderivate.

Thiophenderivate, insbesondere solche, die in 3- und/oder 4-Stellung über ein Sauerstoffatom substituiert sind, sind geeignete Ausgangsmaterialien zur Herstellung elektrisch leitfähiger Polythiophene. Zur Erzielung hoher elektrischer Leitfähigkeiten spielt hierbei die Reinheit der Thiophenderivate eine entscheidende Rolle. Als herkömmliche Reinigungsverfahren für Thiophenderivate wird üblicherweise die Destillation verwendet.

Es hat sich nun aber gezeigt, daß die so gereinigten Thiophene im Laufe der Lagerung zu Farbveränderungen und/oder zur Bildung von unerwünschten Nebenkomponenten wie z.B. zur Dimerbildung neigen. Dies führt zur erheblichen Verschlechterung der Eigenschaften der daraus hergestellten Polythiophene.

Es bestand somit weiterhin Bedarf an einem Verfahren zur Stabilisierung solcher Thiophenderivate, um die vorangehend beschriebenen Farbveränderungen bzw. Bildung von Nebenkomponenten zu vermeiden und damit Thiophenderivate bereitzustellen, die auch nach Lagerung eine ausreichende Reinheit für die Weiterverarbeitung zu elektrisch leitfähigen Polythiophenen aufweisen.

Somit bestand die Aufgabe der vorliegenden Erfindung darin, ein solches Verfahren zur Herstellung von Thiophenderivaten geeigneter Qualität aufzufinden.

Überraschenderweise wurde jetzt gefunden, dass sich Thiophenderivate der Formel (I) 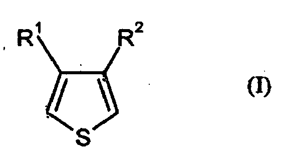 durch Behandlung mit basischen Verbindungen nach der Herstellung und Reinigung, z.B. durch Destillation, stabilisieren lassen und unerwünschte Farbveränderungen und die Bildung von Nebenkomponenten vermieden werden können.

Gegenstand der vorliegenden Erfindung ist somit ein Verfahren zur Stabilisierung von Thiophenderivaten der allgemeinen Formel (I), 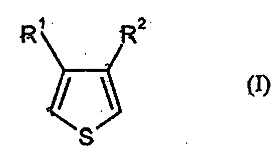 worin
R¹ und R² unabhängig von einander für Wasserstoff, für gegebenenfalls substituierte, gegebenenfalls durch 1 bis 5 Sauerstoff- und/oder Schwefelatome unterbrochene C₁-C₂₀-Alkylgruppe oder C₁-C₂₀-Oxyalkylgruppe oder gemeinsam für eine gegebenenfalls substituierte C₁-C₂₀-Dioxyalkylen- oder C₁-C₂₀-Dioxyarylengruppe stehen,
dadurch gekennzeichnet, dass die Thiophenderivate mit basischen Verbindungen behandelt werden.

Als basische Verbindungen im Rahmen der Erfindung sind solche zu verstehen, deren 10 Gew.-%ige Aufschlämmung oder Lösung in Wasser einen pH-Wert von größer 7, bevorzugt größer 8 aufweist. Der pH-Wert wird gemessen bei 20°C. Bevorzugt werden als basische Verbindungen Feststoffe eingesetzt, die in den zu stabilisierenden Thiophenen unlöslich oder nur sehr schlecht löslich sind.

Bevorzugt werden für das erfindungsgemäße Verfahren als basische Verbindungen Alkali- oder Erdalkalihydroxide, Alkali- oder Erdalkalialumosilikate, Hauptgruppenmetalloxide oder Übergangsmetalloxide oder Ionenaustauscherharze verwendet.

Als geeignete Alkali und Erdalkalihydroxide seien beispielsweise Lithiumhydroxid, Natriumhydroxid, Kaliumhydroxid, Calciumhydroxid, Magnesiumhydroxid oder Bariumhydroxid genannt.

Als geeignete Hauptgruppenmetalloxide seien beispielsweise Calciumoxid, Magnesiumoxid, Bariumoxid, Aluminiumoxid, Wismutoxid, Zinnoxid, Bleioxid oder Zinkoxid, wobei Zinkoxid als Oxid eines Übergangsmetalls aufgrund seiner Hauptgruppenoxidähnlichen Eigenschaften dieser Gruppe zugeordnet wird. Unter geeigneten Hauptgruppenmetalloxiden sind auch gegebenenfalls deren Oxidhydroxide mit umfasst.

Als Alkali- bzw. Erdalkalialumosilikate seien beispielsweise solche der allgemeinen Formel (A),

M_{2/z}·Al₂O₃·xSiO₂·yH₂O (A)

worin
- M: ein ein- oder mehrwertiges Alkali- oder Eradalkalimetall, H oder NH₄,
- Z: die Wertigkeit des Kations,
- x: für eine Zahl von 1,8 bis 12 und
- y: für eine Zahl von 0 bis 8, wobei dies nicht nur ganze Zahlen einschließt, stehen.

Als Übergangsmetalloxide seien beispielhaft die der Metalle Eisen, Kupfer, Nickel, Kobalt, Mangan, Molybdän, Niob, Titan, Tantal, Wolfram oder gegebenenfalls deren Oxidhydroxide genannt.

Als Ionenaustauscherharze seien solche beispielhaft genannt, die funktionelle basische Gruppen, wie beispielsweise primäre, sekundäre oder tertiäre Amingruppen bzw. quartäre Ammoniumgruppen aufweisen. Nach Art und Kombination der funktionellen Gruppen können die Ionentauscher in ihrer Basizität variieren. Beispielsweise enthalten stark basische Ionenaustauscher gewöhnlich quartäre Ammoniumgruppen, während schwach basische Ionenaustauscher häufig die weniger basischen primären, sekundären und/oder tertiären Amingruppen tragen. Jedoch sind zwischen stark und schwach basischen Ionenaustauschern auch beliebige Mischformen bekannt.

Besonders bevorzugt werden als basische Verbindungen im Rahmen der Erfindung Calciumoxid, Magnesiumoxid, Bariumoxid, Aluminiumoxid oder gegebenenfalls deren Oxidhydroxide verwendet.

Die basischen Verbindungen können in kommerziell erhältlicher Form, d.h. Pulver, Granulat, Perlen etc. eingesetzt werden und bedürfen keiner weiteren Aufreinigung oder Behandlung.

Die basischen Verbindungen können gegebenenfalls in Kombination mit weiteren absorptiv wirkenden Verbindungen wie z.B. Aktivkohle verwendet werden. Bevorzugt wird neutrale oder basisch behandelte Aktivkohle verwendet.

Thiophenderivate sind im Rahmen der Erfindung bevorzugt solche der allgemeinen Formel (II), 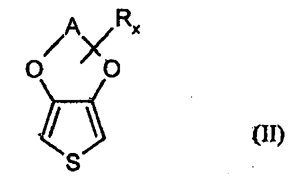 worin
- A: für einen gegebenenfalls substituierten C₁-C₅-Alkylenrest oder einen C₁-C₁₂-Arylenrest, bevorzugt für einen gegebenenfalls substituierten C₂-C₃-Alkylenrest, steht,
- R: für einen linearen oder verzweigten, gegebenenfalls substituierten C₁-C₁₈-Alkylrest, bevorzugt linearen oder verzweigten, gegebenenfalls substituierten C₁-C₁₄-Alkylrest, einen gegebenenfalls substituierten C₅-C₁₂-Cycloalkylrest, einen gegebenenfalls substituierten C₆-C₁₄-Arylrest, einen gegebenenfalls substituierten C₇-C₁₈-Aralkylrest, einen gegebenenfalls substituierten C₁-C₄-Hydroxyalkylrest, bevorzugt gegebenenfalls substituierten C₁-C₂-Hydroxyalkylrest, oder einen Hydroxylrest steht,
- x: für eine ganze Zahl von 0 bis 8 steht, bevorzugt von 0 bis 6, besonders bevorzugt für 0 oder 1 steht und
für den Fall, dass mehrere Reste R an A gebunden sind, diese gleich oder unterschiedlich sein können.

Die allgemeine Formel (II) ist so zu verstehen, dass der Substituent R x-mal an den Alkylen- oder Arylenrest A gebunden sein kann.

Besonders bevorzugt sind Thiophenderivate im Rahmen der Erfindung solche der allgemeinen Formel (IIa) 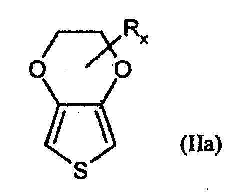 worin R und x die für die allgemeine Formel (II) genannte Bedeutung haben.

In einer bevorzugten Ausführungsform der Erfindung handelt es sich bei dem Thiophenderivat um 3,4-Ethylendioxythiophen.

C₁-C₅-Alkylenreste A sind im Rahmen der Erfindung Methylen, Ethylen, n-Propylen, n-Butylen oder n-Pentylen. C₁-C₁₂-Arylenreste A können im Rahmen der Erfindung beispielsweise Phenylen, Naphthylen, Benzyliden oder Antlaracenyliden sein. C₁-C₁₈-Alkyl steht im Rahmen der Erfindung für lineare oder verzweigte C₁-C₁₈-Alkylreste wie beispielsweise Methyl, Ethyl, n- oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl, n-Tetradecyl, n-Hexadecyl oder n-Octadecyl. C₁-C₂₀-Alkylgruppen umfassen darüber hinaus beispielsweise n-Nonadecyl und n-Eicosyl. C₅-C₁₂-Cycloalkyl steht im Rahmen der Erfindung für C₅-C₁₂-Cycloalkylreste wie beispielsweise Cyclopentyl, Cyclohexyl, Cycloheptyl, Cyclooctyl, Cyclononyl oder Cyclodecyl, C₅-C₁₄-Aryl für C₅-C₁₄-Arylreste wie beispielsweise Phenyl oder Naphthyl, und C₇-C₁₈-Aralkyl für C₇-C₁₈-Aralkylreste wie beispielsweise Benzyl, o-, m-, p-Tolyl, 2,3-, 2,4-, 2,5-, 2,6-, 3,4-, 3,5-Xylyl oder Mesityl. C₁-C₂₀-Oxyalkyl steht im Rahmen der Erfindung für C₁-C₂₀-Oxyalkylreste wie beispielsweise Methoxy, Ethoxy, n- oder iso-Propoxy, n-, iso-, sec oder tert-Butoxy, n-Pentyloxy, 1-Methylbutyloxy, 2-Methylbutyloxy, 3-Methylbutyloxy, 1-Ethylpropyloxy, 1,1-Dimethylpropyloxy, 1,2-Dimethylpropyloxy, 2,2-Dimethylpropyloxy, n-Hexyloxy, n-Heptyloxy, n-Octyloxy, 2-Ethylhexyloxy, n-Nonyloxy, n-Decyloxy, n-Undecyloxy, n-Dodecyloxy, n-Tridecyloxy, n-Tetradecyloxy, n-Hexadecyloxy, n-Octadecyloxy, n- Nonadecyloxy oder n-Eicosyloxy. Die vorangehende Aufzählung dient der beispielhaften Erläuterung der Erfindung und ist nicht als abschließend zu betrachten.

Als gegebenenfalls weitere Substituenten der Alkylen- oder Arylenreste A kommen zahlreiche organische Gruppen in Frage, beispielsweise Alkyl-, Cycloalkyl-, Aryl-, Halogen-, Ether-, Thioether-, Disulfid-, Sulfoxid-, Sulfon-, Sulfonat-, Amino-, Aldehyd-, Keto-, Carbonsäureester-, Carbonsäure-, Carbonat-, Carboxylat-, Cyano-, Alkylsilan- und Alkoxysilangruppen sowie Carboxylamidgruppen.

Sofern das Thiophenderivat ein oder mehrere Stereozentren aufweist, kann es sich bei dem Thiophenderivat um ein Racemat, eine enantiomerenreine oder diastereomerenreine Verbindung oder um eine enantiomerenangereicherte oder diastereomerenangereicherte Verbindung handeln. Unter einer entantiomerenangereicherten Verbindung ist eine Verbindung mit einem Enantiomerenüberschuss (ee) von mehr als 50 % zu verstehen. Unter einer diastereomerenangereicherten Verbindung ist eine Verbindung mit einem Diastereomerenüberschuss (de) von mehr als 30 % zu verstehen. Erfindungsgemäß kann es sich jedoch auch um eine beliebige Mischung aus Diastereomeren handeln.

Die Thiophenederivate der allgemeinen Formeln (I), (II) oder (IIa) sind nach dem Fachmann bekannten Verfahren herstellbar. Beispielsweise ist ein solches Herstellverfahren in EP-A 1 142 888 beschrieben.

Bezogen auf das Gewicht des Thiophenderivats werden 0,01 bis 50 Gew.-%, bevorzugt 0,1 bis 10 Gew.-%, besonders bevorzugt 0,5 bis 5 Gew.-% der basischen Verbindung oder des Gemisches aus basischer Verbindung und weiterer absorptiv wirkender Verbindung verwendet.

Die Behandlung der Thiophenderivate mit den basischen Verbindungen gegebenenfalls im Gemisch mit der weiteren absorptiven Verbindung kann beispielsweise durch Zugabe dieser Verbindungen zu den Thiophenderivaten, Rühren und anschließendem Abfiltrieren der basischen Verbindung und gegebenenfalls absorptiver Verbindung erfolgen.

Die Behandlung kann auch in der Weise erfolgen, dass die basische Verbindung gegebenenfalls im Gemisch mit der weiteren absorptiven Verbindung in eine geeignete Filtereinheit oder Säule gefüllt wird und anschließend das Thiophenderivat über die basische Verbindung und gegebenenfalls weitere absorptive Verbindung geleitet wird. Dieses Verfahren hat beispielsweise großtechnisch den Vorteil, dass die zusätzliche Filtration eingespart wird.

Die Dauer der Behandlung der Thiophenderivate kann von wenigen Sekunden bis zu mehreren Stunden dauern.

Die Behandlung der Thiophenderivate mit den basischen Verbindungen kann bei Temperaturen von -10°C bis 150°C erfolgen. Bevorzugt erfolgt die Behandlung bei Raumtemperatur.

Die mit dem erfindungsgemäßen Verfahren stabilisierten Thiophenderivate neigen deutlich weniger zu Farbveränderungen und/oder Bildung von Nebenkomponenten und weisen daher eine deutlich höhere Lagerstabiliät auf. Sie unterscheiden sich von den nicht gemäß dem erfindungsverfahren stabilisierten Thiophenderivaten significant in ihren Eigenschaften.

Die folgenden Beispiele dienen der beispielhaften Erläuterung der Erfindung und sind nicht als Beschränkung aufzufassen.

### Beispiele

### Beispiel 1:

In eine durch eine Glasfritte unten verschlossene Glassäule (Länge 20 cm, Durchmesser 1 cm) wurden 2 g basisches Aluminiumoxid (Fa. Aldrich, pH-Wert in wässriger Lösung 9,5 + - 0,5, Partikelgröße ca. 150 mesh) gefüllt. Über das Aluminiumoxid wurden 50 g frisch destilliertes 3,4-Ethylendioxythiophen gegeben. Das behandelte 3,4-Etlaylendioxytlainplaen wurde in eine 100 ml Glasflasche gefüllt.

Des Weiteren wurden 50 g des frisch destillierten 3,4-Ethylendioxythiophens ohne die erfindungsgemäße Behandlung mit dem basischen Aluminiumoxid in eine 100 ml Glasflasche gefüllt. Beide Proben wurden unter gleichen Bedingungen gelagert.

Anschließend wurde die Farbveränderungen der behandelten Probe im Vergleich zu der nicht behandelten Probe im Laufe der Zeit beobachtet.

Nach 4 Wochen war die nicht behandelte Probe deutlich stärker gelb verfärbt als die mit Aluminiumoxid behandelte Probe.

## Patentansprüche

1. Verfahren zur Stabilisierung von Thiophenderivaten der allgemeinen Formel (I), 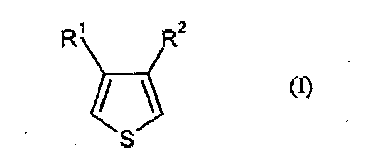 worin
R¹ und R² unabhängig von einander für Wasserstoff, für gegebenenfalls substituierte, gegebenenfalls durch 1 bis 5 Sauerstoff- und/oder Schwefelatome unterbrochene C₁-C₂₀-Alkylgruppe oder C₁-C₂₀-Oxyalkylgruppe oder gemeinsam für eine gegebenenfalls substituierte C₁-C₂₀-Dioxyalkylen- oder C₁-C₂₀-Dioxyarylengrupe stehen,
**dadurch gekennzeichnet, dass** die Thiophenderivate mit basischen Verbindungen behandelt werden.

2. Verfahren gemäß Anspruch 1, **dadurch gekennzeichnet, dass** als basische Verbindungen Alkali- oder Erdalkalihydroxide, Alkali- oder Erdalkalialumosilikate, Hauptgruppenmetalloxide oder Übergangsmetalloxide oder Ionenaustauscherhaze verwendet werden.

3. Verfahren gemäß Anspruch 1 oder 2, **dadurch gekennzeichnet, dass** als basische Verbindungen Calciumoxid, Magnesiumoxid, Bariumoxid, Aluminiumoxid oder gegebenenfalls deren Oxidhydroxide verwendet werden.

4. Verfahren gemäß wenigstens einem der Ansprüche 1 bis 3, **dadurch gekennzeichnet, dass** zusätzlich zu den basischen Verbindungen absorptive wirkende Verbindungen, bevorzugt neutrale oder basische Aktivkohle eingesetzt werden.

5. Verfahren gemäß wenigstens einem der Ansprüche 1 bis 4, **dadurch gekennzeichnet, dass** es sich bei den Thiophenderivaten um solche der allgemeinen Formel (II) handelt, 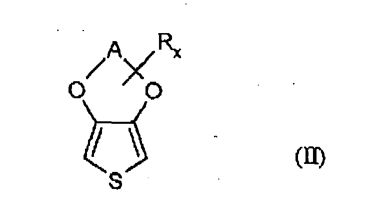 worin
A für einen gegebenenfalls substituierten C₁-C₅-Alkylenrest oder einen C₁-C₁₂- Arylenrest, bevorzugt für einen gegebenenfalls substituierten C₂-C₃-Alkylenrest, steht,
R für einen linearen oder verzweigten, gegebenenfalls substituierten C₁-C₁₈-Alkylrest, bevorzugt linearen oder verzweigten, gegebenenfalls substituierten C₁-C₁₄-Alkyl- rest, einen gegebenenfalls substituierten C₅-C₁₂-Cycloalkylrest, einen gegebenen- falls substituierten C₆-C₁₄-Arylrest, einen gegebenenfalls substituierten C₇-C₁₈- Aralkylrest, einen gegebenenfalls substituierten C₁-C₄-Hydroxyalkylrest be- vorzugt gegebenenfalls substituierten C₁-C₂-Hydroxyalkylrest, oder einen Hydroxylrest steht,
x für eine ganze Zahl von 0 bis 8 steht, bevorzugt von 0 bis 6, besonders bevorzugt für 0 oder 1 steht und
für den Fall, dass mehrere Reste R an A gebunden sind, diese gleich oder unterschiedlich sein können.

6. Verfahren gemäß wenigstens einem der Ansprüche 1 bis 5, **dadurch gekennzeichnet, dass** es sich bei dem Thiophenderivaten allgemeinen Formel (IIa), 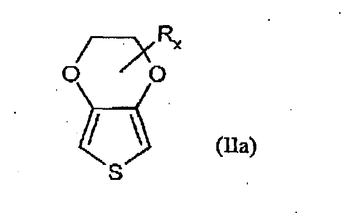 worin R und x die in Anspruch 5 genannte Bedeutung haben.

7. Verfahren gemäß wenigstens einem der Ansprüche 1 bis 6, **dadurch gekennzeichnet, dass** es sich bei dem Thiophenderivat um 3,4-Ethylendioxythiophen handelt.

## Claims

1. Method for stabilizing thiophene derivatives of the general formula (I) 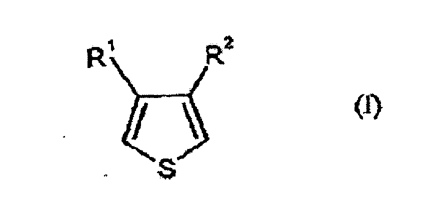 wherein
R¹ and R² independently of each other represent hydrogen, an optionally substituted C₁-C₂₀-alkyl group or C₁-C₂₀-oxyalkyl group which is optionally interrupted by 1 to 5 oxygen and/or sulphur atoms, or together represent an optionally substituted C₁-C₂₀-dioxyalkylene or C₁-C₂₀-dioxyarylene group,
**characterized in that** the thiophene derivatives are treated with basic compounds.

2. Method according to claim 1, **characterized in that** alkali metal or alkaline earth metal hydroxides, alkali metal or alkaline earth metal alumosilicates, main group metal oxides or transition metal oxides or ion exchanger resins are used as the basic compounds.

3. Method according to claim 1 or 2, **characterized in that** calcium oxide, magnesium oxide, barium oxide, aluminium oxide or optionally oxyhydroxides thereof are used as the basic compounds.

4. Method according to at least one of claims 1 to 3, **characterized in that** in addition to the basic compounds, compounds having an absorptive action, preferably neutral or basic active charcoals, are employed.

5. Method according to at least one of claims 1 to 4, **characterized in that** the thiophene derivatives are those of the general formula (II) 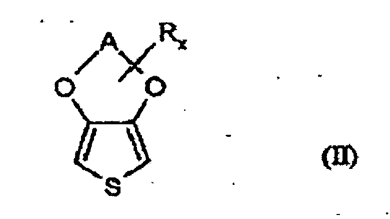 wherein
A represents an optionally substituted C₁-C₅-alkylene radical or a C₁-C₁₂-arylene radical, preferably an optionally substituted C₂-C₃-alkylene radical,
R represents a linear or branched, optionally substituted C₁-C₁₈-alkyl radical, pre- ferably linear or branched, optionally substituted C₁-C₁₄-alkyl radical, an op- tionally substituted C₅-C₁₂-cycloalkyl radical, an optionally substituted C₆-C₁₄- aryl radical, an optionally substituted C₇-C₁₈-aralkyl radical, an optionally subs- tituted C₁-C₄-hydroxyalkyl radical, preferably optionally substituted C₁-C₂- hydroxyalkyl radical, or a hydroxyl radical,
x represents an integer from 0 to 8, preferably from 0 to 6, and particularly pre- ferably represents 0 or 1 and
in the case where several radicals R are bonded to A, these can be identical or different.

6. Method according to at least one of claims 1 to 5, **characterized in that** the thiophene derivatives have general formula (IIa) 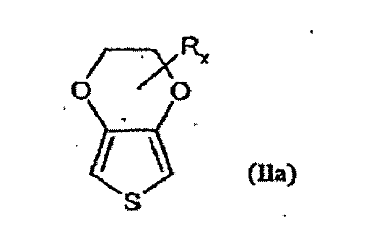 wherein R and x have the meaning given in claim 5.

7. Method according to at least one of claims 1 to 6, **characterized in that** the thiophene derivative is 3,4-ethylenedioxythiophene.

## Revendications

1. Procédé pour la stabilisation de dérivés du thiophène de la formule générale (I), 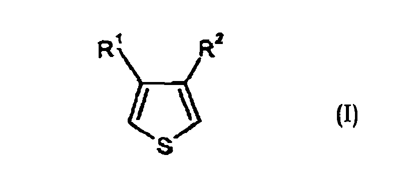 dans laquelle
R¹ et R² représentent indépendamment l'un de l'autre un hydrogène, un groupe alkyle en C₁ à C₂₀ ou un groupe oxyalkyle en C₁ à C₂₀, substitué le cas échéant, interrompus le cas échéant par 1 à 5 atomes d'oxygène et/ou de soufre, ou ensemble un groupe dioxyalkylène en C₁ à C₂₀ ou un groupe dioxyarylène en C₁ à C₂₀ substitué le cas échéant,
**caractérisé en ce que** les dérivés du thiophène sont traités avec des composés basiques.

2. Procédé selon la revendication 1, **caractérisé en ce que** l'on utilise comme composés basiques des hydroxydes alcalins ou alcalinoterreux, des alumosilicates alcalins ou alcalinoterreux, des oxydes de métaux du groupe principal ou des oxydes de métaux de transition ou des résines échangeuses d'ions.

3. Procédé selon la revendication 1 ou 2, **caractérisé en ce que** l'on utilise comme composés basiques de l'oxyde de calcium, de l'oxyde de magnésium, de l'oxyde de baryum, de l'oxyde d'aluminium ou le cas échéant leurs hydroxy-oxydes.

4. Procédé selon l'une au moins des revendications 1 à 3, **caractérisé en ce qu'**en plus des composés basiques, on utilise des composés à action absorbante, de préférence du charbon actif neutre ou basique.

5. Procédé selon l'une au moins des revendications 1 à 4, **caractérisé en ce qu'**il s'agit en fait de dérivés du thiophène de dérivés de la formule générale (II), 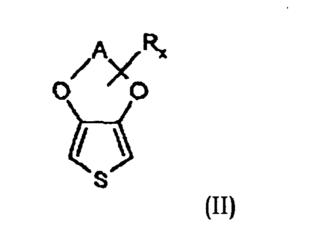 dans laquelle
A représente un radical alkylène en C₁ à C₅, substitué le cas échéant, ou un radical arylène en C₁ à C₁₂, de préférence un radical alkylène en C₂ - C₃, substitué le cas échéant,
R représente un radical alkyle en C₁ à C₁₈ linéaire ou ramifié, substitué le cas échéant, de préférence un radical alkyle en C₁ à C₁₄ linéaire ou ramifié, substitué le cas échéant, un radical cycloalkyle en C₅ à C₁₂, substitué le cas échéant, un radical aryle en C₆ à C₁₄ substitué le cas échéant, un radical aralkyle en C₇ à C₁₈ substitué le cas échéant, un radical hydroxyalkyle en C₁ à C₄ substitué le cas échéant, de préférence un radical hydroxyalkyle en C₁ - C₂ substitué le cas échéant, ou un radical hydroxyle,
x représente un nombre entier de 0 à 8, de préférence de 0 à 6, de façon particulièrement préférée 0 ou 1, et,
pour le cas où plusieurs radicaux R seraient liés à A, ceux-ci peuvent être identiques ou différents.

6. Procédé selon l'une au moins des revendications 1 à 5, **caractérisé en ce que** le dérivé du thiophène est de la formule générale (IIa), 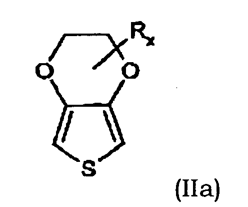 dans laquelle R et x ont les significations énoncées dans la revendication 5.

7. Procédé selon l'une au moins des revendications 1 à 6, **caractérisé en ce qu'**il s'agit en fait de dérivé du thiophène de 3,4-éthylènedioxythiophène.
